# EUROPEAN PATENT SPECIFICATION

(11) **EP 3 272 380 B1**
(45) Date of publication and mention of the grant of the patent: **29.07.2020**
(21) Application number: 17156086.5
(22) Date of filing: 14.02.2017
(51) Int. Cl.: A61M 15/00

(54) **HANDHELD NEBULIZER**
HANDHALTBARER ZERSTÄUBER
NÉBULISEUR PORTATIF

(30) Priority: 18.07.2016 CN 201610564979
(43) Date of publication of application: 24.01.2018
(73) Proprietor: Outstanding Healthcare Company Limited, Hong Kong (CN)
(72) Inventor: ZHENG, Ze Guang, Hung Hom (HK); FU, Kwok Fu, Hung Hom (HK); XU, Jie Bing, Hung Hom (HK)
(74) Representative: Ward, David Ian

(56) References cited:
- EP-A1- 2 987 520
- WO-A1-2015/117702
- US-A1- 2005 199 236
- US-A1- 2006 201 501
- US-A1- 2009 159 719
- US-A1- 2011 259 324
- US-A1- 2012 285 446
- US-A1- 2012 285 447
- US-A1- 2012 291 777
- US-B1- 6 435 175

## Description

### TECHNICAL FIELD

The present invention relates to a handheld nebulizer, in particular to a handheld nebulizer having a removable cartridge for administrating nebulized particles to a subject.

### BACKGROUND

Handheld nebulizer is commonly used to turn a liquid medicament or mixture into nebulized particles such as in the form of vapors. A user can then breathe in the nebulized particles. Such a drug delivery approach is generally applied for treating respiratory diseases or symptoms in a subject in need thereof such as a subject suffering from asthma, cough, fever, sore throat, nasal inflammation etc.

However, the existing handheld nebulizers are generally susceptible to contamination by dirt, bacteria and/or unwanted particles and such a contamination may be associated with an increase in health risks. This is because the existing nebulizers generally require refilling of the liquid medicament to a liquid storage chamber through, for example, syringe injection or replacement of a liquid filing container, without thoroughly cleaning the liquid storage chamber. The nebulizing elements affixed in these nebulizers are repeatedly used and these nebulizing elements cannot be thoroughly cleaned, sterilized or replaced. Further, the existing approaches for refilling, cleaning or replacement are limited and sometime require complicated procedures that a user may not be able to operate.

Moreover, the current handheld nebulizers generate a cool mist from liquid medicament in nebulization for a user to inhale. However, since the temperature of the cool mist is lower than body temperature, the user usually feels uncomfortable when administering the mist. The cool mist may also quickly condense in unwanted places leading to contamination and/or further trigger irritation to the respiratory tract and thus the desired therapeutic effect cannot be attained, or may not be efficiently attained.

Accordingly, there remains a need for an improved handheld nebulizer in which the aforesaid shortcomings are mitigated or at least to provide a useful alternative to the trade and public.

US6435175B1 relates to a nebulizer for delivering a drug to a patient and including an electronic control unit, a hand piece connected to the electronic control unit via a cable, and a cartridge detachably engaged with the hand piece.

US2012/285447A1 relates to a nebulizer comprising a head detachably coupled to a body, the head comprising nebulizing means, an air channel and a flow sensor.

### SUMMARY OF THE INVENTION

The present invention provides a handheld nebulizer as defined by appended claim 1. The handheld nebulizer includes a body having a control assembly and an actuation assembly. The control assembly is in electrical communication with the actuation assembly for delivering nebulized particles of a compound to a subject. A nebulizing chamber accommodates a removable cartridge for conducting nebulization. The removable cartridge holds a mixture of a compound to be nebulized. A nebulizing element is affixed or detachably mounted to the removable cartridge, and the removable cartridge is in electrical communication with the control assembly.

The present invention also pertains to a removable cartridge for a handheld nebulizer, including a nebulizing element.

Without intending to be limited by theory, it is believed that the handheld nebulizer of the present invention may address one or more of the problems discussed above. In particular, the handheld nebulizer of the present invention is easy to operate and can substantially prevent/ minimize contamination by unwanted dirt, bacteria and/or particles on the nebulizing element thereby minimizing health risks caused by such a contamination. The heating element, if present, can further help to reduce undesired irritations caused by the inhalation of cool air or nebulized particles.

It has been found that the handheld nebulizer and the removable cartridge of the present invention are exceptionally suitable for large scale production. The provision of the identification element allows a single handheld nebulizer to correctly and automatically identify various cartridges holding different compounds for carrying out the respective nebulization. This in turn reduces patient error and the chance of improper administration and/or dosages of medicines. In addition, the cartridge used herein may be quick, easy, and inexpensive to produce and may be disposable, which further reduces the chance of contamination.

### BREIF DESCRIPTION OF THE DRAWINGS

Embodiments of the present invention will now be described, by way of example, with reference to the accompanying drawings. In the drawings, identical components/elements are denoted with the identical numerical reference, wherein like numerical references refers to the similar elements. In the present application, the numerical references of similar or identical components in different embodiments (such as 100, 200) may have a number difference of 100 so as to indicate their similarity
Fig. 1 illustrates an embodiment of a handheld nebulizer according to the present invention.
Fig. 2 shows the handheld nebulizer of Fig. 1 with the cover and a part of the housing of the nebulizing chamber removed.
Fig. 3A to Fig. 3G show different views of an embodiment of a removable cartridge according to the present invention; wherein Fig. 3A shows the perspective view, Fig. 3B shows the front view, Fig. 3C shows the rear view, Fig. 3D shows the top view, Fig. 3E shows the bottom view, Fig. 3F shows the right side view and Fig. 3G shows the left side view.
Fig. 4 illustrates an embodiment of a handheld nebulizer according to the present invention.
Fig. 5 illustrates an embodiment of a handheld nebulizer according to the present invention.
Fig. 6 is a diagram showing the actuation assembly applied in the handheld nebulizer of Fig. 5 having an infrared sensor and a movable block.

The drawings herein are for illustrative purposes only and may not be drawn to scale.

### DETAILED DESCRIPTION OF THE INVENTION

The present invention provides a handheld nebulizer including a body having a control assembly and an actuation assembly. The control assembly is in electrical communication with the actuation assembly for delivering nebulized particles of a compound to a subject. A nebulizing chamber accommodates a removable cartridge for conducting nebulization. The removable cartridge holds a mixture of a compound to be nebulized, wherein a nebulizing element is affixed or detachably mounted to the removable cartridge, and wherein the removable cartridge is in electrical communication with the control assembly.

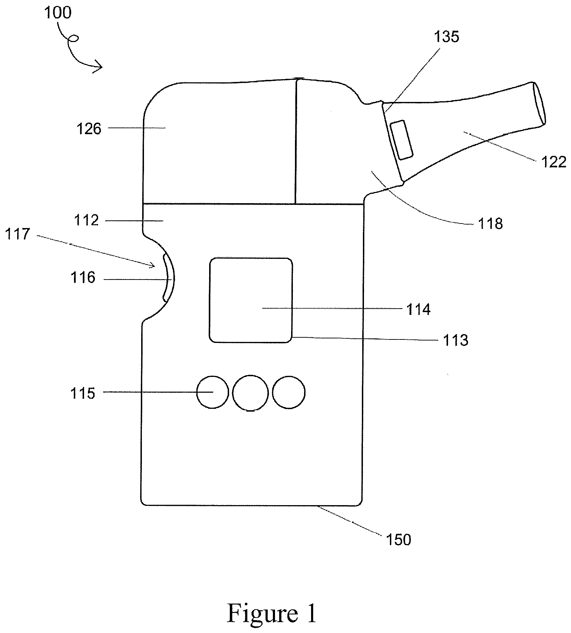

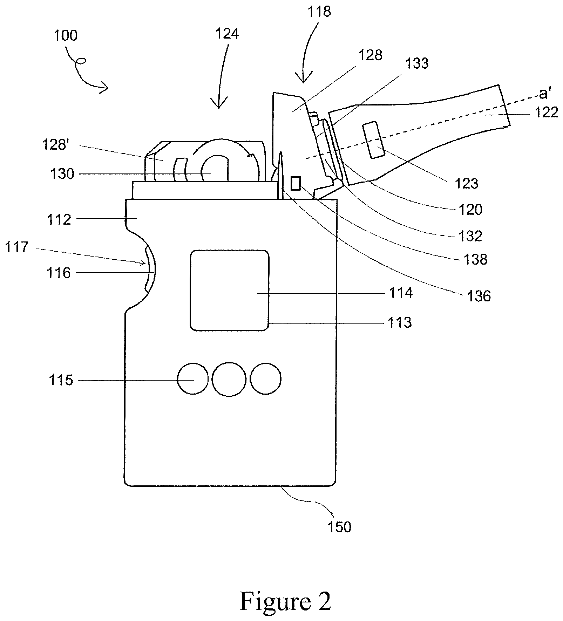

Fig. 1 and 2 show a handheld nebulizer according to an embodiment of the present invention. The handheld nebulizer **100** includes a body **112** which is arranged with a control assembly **113** having a display screen **114** and input buttons **115** on one surface of the body **112** for operating the handheld nebulizer **100.** The body **112** is also provided with an actuation assembly **117** in connection with the control assembly **113**, the actuation assembly **117** includes an actuation element such as an actuation button **116** on a side surface of the body **112** to enable a user to actuate the nebulization for use.

A nebulizing chamber **118** accommodating a removable cartridge **128** is provided above the body **112.** The nebulizing chamber **118** has an outlet **120** which is in fluid communication with a guiding nozzle **122** coupled to the nebulizing chamber **118.** The guiding nozzle **122** extends outwardly from the nebulizing chamber **118** and has two opposite holes **123** on its wall for guiding the flow of nebulized particles. The arrangement of the holes **123** on the guiding nozzle **122** enhances the flow of the nebulized particles when the user inhales, i.e. the suction force generated by the user draws the surrounding air to enter from the holes **123** and provides an additional force for moving the nebulizer particles towards the user in a more efficient manner. Preferably, the direction of the flow of the nebulized particles leaving the outlet **120** is in parallel with the extension direction of the guiding nozzle **122**, i.e. along the axis a'.

Next to the nebulizing chamber **118** is a storage chamber **124** provided with a cover **126.** In Figs. 1 and 2, the storage chamber **124** is optionally-arranged to accommodate an additional removable cartridge **128**' for spare use. The additional removable cartridge allows the user to quickly replace a depleted cartridge with a new and optionally prefilled one. Such an arrangement is particularly advantageous in that it enables the user to easily replace with a new one when he/she is travelling and cannot access to the stock at home. Such an arrangement also avoids complicated replacement steps and reduces the potential for contamination.

With reference to Fig. 2, a removable cartridge **128** is fitted into the nebulizing chamber **118** for nebulization. The removable cartridge **128** is typically prefilled with a mixture of a compound to be nebulized through an opening **130** and is provided with a nebulizing element **132** to cover the opening **130.** In this embodiment, the nebulizing element **132** is arranged with a seal ring **133** and is detachably mounted on the removable cartridge **128.** Before being fitted in the nebulizing chamber **118**, the removable cartridge **128** is optionally additionally protected by a thin membrane (not shown) to prevent the nebulizing element from contacting with any dirt or contamination before use. Alternatively, the nebulizing element **132** may be permanently affixed to the removable cartridge **128** by an adhesive, a fastener, etc., for example, the nebulizing element **132** may be attached to the removable cartridge **128** or fastened by a clip and/or screws. In this embodiment, the nebulizing element **132** includes an ultrasonic atomizer for nebulizing the mixture. Other alternative atomizers known in the technical field may be applied.

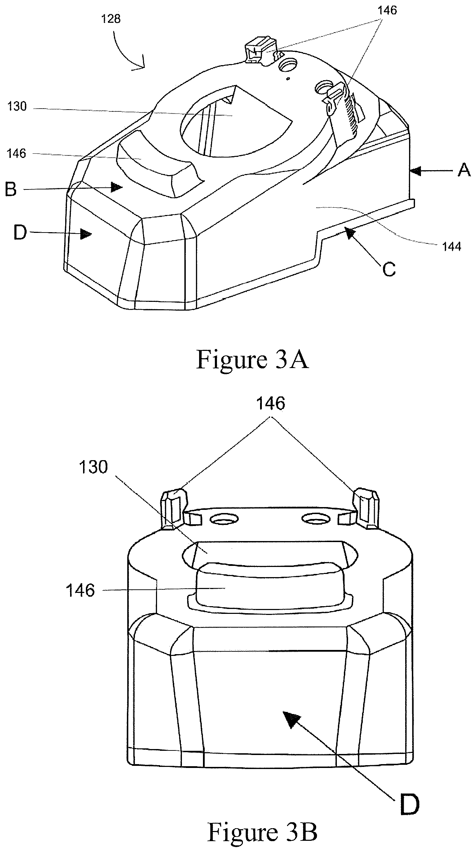

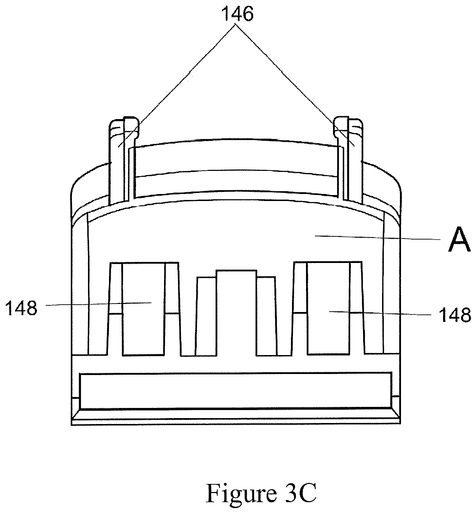

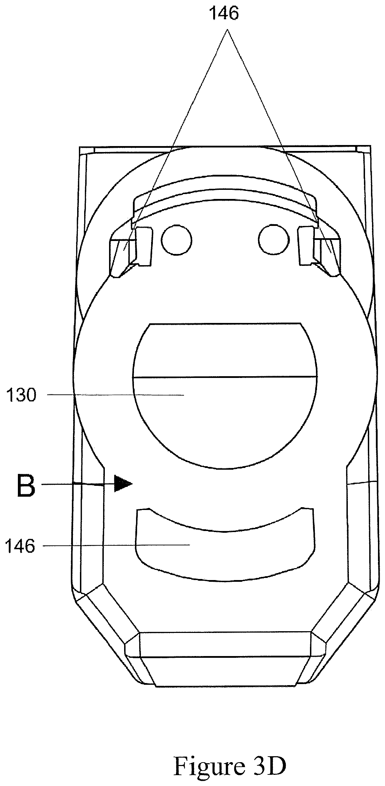

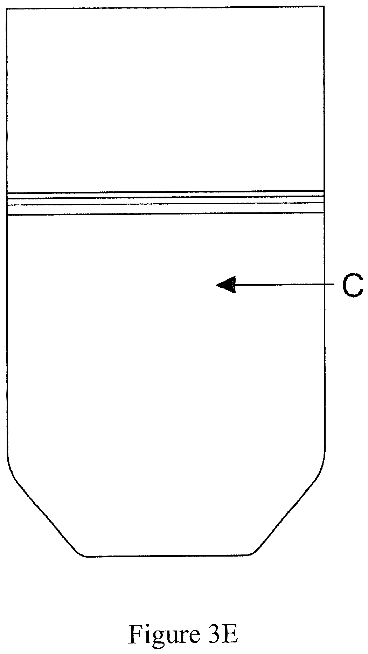

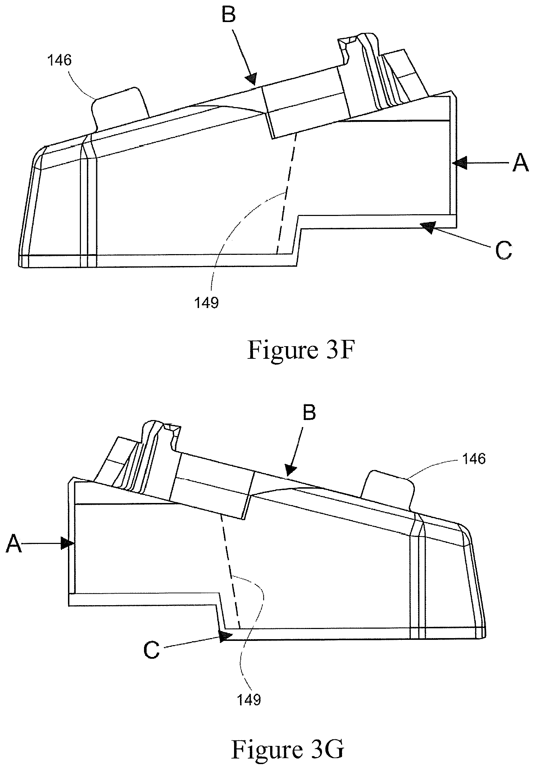

When the removable cartridge **128** is fitted into the nebulizing chamber **118**, it is in electrical communication with the control assembly **113.** In particular, the removable cartridge **128** may be provided with one or more electronic contacts (see Fig. 3 at **148**) to interact with the control assembly **113** and thus when the nebulizer is actuated, the nebulizing element **132** is triggered to nebulize the mixture so as to produce nebulized particles. Alternatively, the removable cartridge **128** may be provided with one or more physical contacts and/or electrical chips to interact with the control assembly **113.**

Preferably, in an embodiment herein, the nebulized particles have an average size of about or less than 5µm, for example from about 1 µm to about 5 µm; or less than about 1 µm; or about 1 µm; or about 2 µm; or about 3 µm; or about 4 µm; or about 5 µm; or from about 1.5 µm to about 4.5 µm; or from about 2.5 µm to about 4 µm; or any combination of the above values. The skilled person is aware of suitable methods for determining the average size of particles, or called the average mass median aerodynamic diameter of particle. For example, the size of the particle is determined by the method described in the European Standard EN 13544-1:2007+A1:2009(E) entitled: Respiratory therapy equipment - Part 1: Nebulizing systems and their components. In particular, the particle sizing method is described in Annex BB and CC.

The nebulizing chamber **118** further includes an output seal ring **135** which is adapted to couple with the removable cartridge **128.** The output seal ring **135** abuts the nebulizing element **132** and holds the removable cartridge **128** in place and/or avoid leakage of any mixture or nebulized particles in between the removable cartridge **128** and the nebulizing chamber **118.** The seal ring **135** also helps to reduce the chance of contamination and may prevent back flow of the nebulized particles into the removable cartridge **128** or nebulizing chamber **118.**

When using the handheld nebulizer **100,** a user can quickly and easily fit a removable cartridge **128** into the nebulizing chamber **118** and switch on the handheld nebulizer. The display screen **114** then shows parameters of the nebulization and/or working modes of nebulization. Parameters of the nebulization may include, for example, the length of time of the nebulization process, the time interval, temperature, the speed of the nebulization, etc. The working modes of nebulization typically refer to preset nebulization modes/patterns. The user can select a suitable mode from various available choices for conducting the nebulization. Accordingly, the user may select the predetermined setting or adjust parameters of the nebulization through the input buttons **115** according to the user's actual requirements. When a desired working mode is selected, the user holds the handheld nebulizer **100**, typically directs the guiding nozzle **122** towards his/her mouth and/or nose, and actuates the nebulization by pressing the actuation button **116.** The nebulizing element of the removable cartridge **128** is then triggered to nebulize the mixture and produce nebulized particles. The nebulized particles travel along the guiding nozzle **122** and are inhaled by the user. The nebulization may continue until the actuation button **116** is released. Alternatively, the user may select a working mode that follows a user's breathing pattern. In such a case, the nebulization may follow the user's breathing pattern.

In the present invention, the handheld nebulizer **100** further includes a heating element **136.** The heating element **136** may be arranged in the nebulizing chamber **118** and/or attached on/in the removable cartridge **128** for heating the mixture in the removable cartridge **128** before the nebulization. Without intending to be limited by theory, it is believed that a heating element is particularly advantageous to provide the nebulized particles with a suitable average temperature for the user to inhale. The user can then inhale warm nebulized particles to relieve any symptoms or illness.

Preferably, the nebulized particles delivered after the heating and nebulization have an average temperature of from about 20°C to about 40°C, from about 23°C to about 37°C, or from about 25°C to about 35°C. In one embodiment, the average temperature is from about 25°C to about 35°C, 25°C, 26°C, 27°C, 28°C, 29°C, 30°C, 31°C, 32°C, 33°C, 34 °C or 35°C. The skilled person is aware of suitable means to determine the average temperature of the nebulized particles for example using a negative temperature coefficient type sensor, and would appreciate that different compounds may be nebulized and delivered at different temperature according to their physicochemical properties. Average temperature may be obtained by repeating the measurement for at least three times. The skilled person appreciates that various heating elements may be applied to the present invention to heat the mixture. For example, the heating element may be selected from the group consisting of a heating coil, a heating plate, a copper tube, and a combination thereof.

In a further embodiment of the present invention, the handheld nebulizer **100** further includes an identification element **138** on the removable cartridge **128.** The identification element **138** can be recognized by the control assembly **113** to determine the identity of the removable cartridge **128.** The identity consists of one or more pieces of information, for example, components of the mixture loaded in the removable cartridge **128**, i.e. types of compound, carrier (e.g. solvent) and the like, instructions for use, and/or the application of the mixture.

In Fig. 1, the identification element **138** is a RFID tag, but one skilled in the art understands that the identification element may alternatively be a designed structure on the removable cartridge integrated in to the cartridge, a separate element attached to the cartridge, etc. The identification element may be selected from the group consisting of a RFID tag, an electronic chip card, a printed label, a designed integrated structure and a combination thereof. In the embodiment of Figs. 1-2, the control assembly **113** is configured to recognize the identity of the removable cartridge **128** and adjusts preset parameters of the handheld nebulizer **100** and/or selects a respective preset mode according to the identity of the removable cartridge 128. The control assembly 113 may detect the identity of the removable cartridge 128 directly or indirectly.

It is appreciated that in an embodiment of the present invention, the control assembly may include a display, a timer, a sensor, an input button, a circuit board, a database or a combination thereof; and the actuation element of the actuation assembly may include a switch, button, a sensor or a combination thereof, or any component that can react with the user to deliver an actuation signal. Other possible obvious variations or modifications may also be applied to the present invention.

It is also appreciated that the mixture of a compound to be nebulized may include a medicament, an essential oil, and/or an excipient. The mixture may be a solution, a dispersion, a suspension, or a colloid; or a solution. The mixture may also be a pure compound.

The present invention also pertains to a removable cartridge for a handheld nebulizer. Fig. 3 shows different views of an embodiment of a removable cartridge of the present invention that may be adapted to the handheld nebulizer **100** as described above. As shown in Fig. 3, the removable cartridge **128** includes an opening **130** and a cavity **144** for holding a mixture to be nebulized. The protrusions **146** as shown in the figure are configured to receive a nebulizing element **132** between the opening **130** and the protrusions **146.** The nebulizing element is arranged with a seal ring **133** to connect and abut the protrusions **146**. When the removable cartridge **128** is loaded with the mixture, the nebulizing element **132** is then affixed to or detachably mounted to the removable cartridge **128** to cover the opening **130.**

The removable cartridge **128** is shaped to be fitted in a respective handheld nebulizer. In this embodiment, when the removable cartridge **128** is fitted into a nebulizing chamber **118** having a similar design as shown in Figs. 1 and 2, a surface **A** of the cartridge faces towards the body, i.e. downwards, and a surface **B** faces towards the outlet of the nebulizing chamber. The electrical contacts **148** arranged on the surface **A** are then in contact with the control assembly of the handheld nebulizer. In use, the control assembly (e.g. as shown as **113** in Fig. 1) may send an actuation signal to the nebulizing element **132** through the electrical contacts **148** which is also in electrical connection with the nebulizing element **132.** The step-like surface **C** allows the mixture to flow down towards surface **A** and at the same time keeps the cartridge firmly in the nebulizing chamber. Preferably, the removable cartridge **128** includes an inner wall **149** (as indicated by a dotted line in Fig. 3F and 3G). The inner wall **149** and the surface **B** form an acute angle of less than 90°C. As such, the contact between the mixture and the nebulizing element **132** is maximized for efficient nebulization and to reduce waste. To sum up, the provision of the step-like surface **C**, surface **B** and inner wall **149** improves the efficiency of the nebulization. It is appreciate that other shapes of the cartridge can also be applicable.

In an embodiment herein a heating element and an identification element may be applied in the cartridge of the invention to achieve the desired improved effects.

It is found that, by way of the arrangements and structures of the handheld nebulizer and removable cartridge as discussed above in particular the application of a removable cartridge, the handheld nebulizer substantially reduces contamination of unwanted dirt or particles on the nebulizing element thereby minimizing health risks caused by such a contamination. The handheld nebulizer is also easy to operate, especially when the mixture to be nebulized is completely depleted, a new cartridge is readily available to replace the depleted one. Also, complicated and potentially dangerous procedures such as using a syringe to refill the mixture are thus avoided. Furthermore, and without intending to be limited by theory, it is believed that a handheld nebulizer of the present invention having the heating element can reduce undesirable irritation caused by the inhalation of cool air or nebulized particles, especially in frail or elderly users. It is believed that warmer nebulized particles and/or air is less likely to cause a constriction of the airway of the user during inhalation, and is therefore more easily drawn into the body, for example, the mouth, the nasal passages, the esophagus, the lungs, etc. The inventors believe that this is especially true for those who are used to breathing humid and/or warm air, such as those people who are living in the tropics or from such locations. The user can thus more easily and comfortably inhale relatively warm nebulized particles to relieve any pain or illness. The control assembly which is in communication with the heating element may adjust the heating temperature according to the surrounding temperature as detected by a temperature sensor **150** (see Figs. 1-2). In an embodiment herein, a temperature sensor **150** is provided in the handheld nebulizer. Therefore, the parameters can be easily adjusted according to the change of weather and it does not require many efforts from the user to do so.

Without intending to be limited by the theory it is believed that when the nebulized particles are inhaled by the user with a suitable average temperature in particular with an average temperature close to the body temperature or close to internal body temperature, it is believed that the absorption efficiency of the compound/medicament or the therapeutic efficacy is enhanced.

In addition, a removable cartridge of the present invention having an identification element enables the handheld nebulizer to recognize, for example, what compound is present in the cartridge and which working mode is suitable for said compound. The control assembly may then automatically adjust to a predetermined optimized mode for nebulizing said compound. The provision of identification element is advantageous in that various types of removable cartridge can be manufactured and each of them is assigned with a specific mixture to be nebulized. In other words, one handheld nebulizer can satisfy different types of removable cartridge which hold different mixtures. The identification element also minimizes the incidents caused by the user mistakenly inputting the wrong parameters.

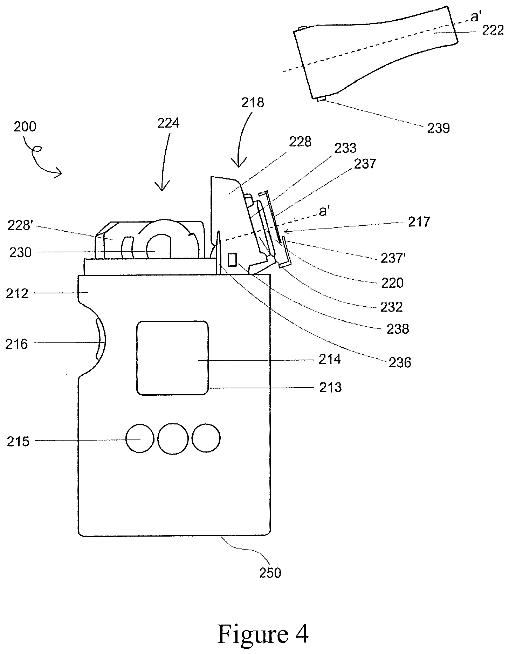

The present invention also provides a handheld nebulizer that can be operated according to a user's breathing, i.e. the handheld nebulizer synchronizes to a user's breathing action, such as their inhalation and exhalation. In the embodiment for Figs. 4-5, the handheld nebulizer includes an actuation assembly which is responsive to a user's breathing. When the handheld nebulizer is in use, an actuation element of the actuation assembly may detect the air current passing through the guiding nozzle and then sends an actuation signal to the control assembly. The nebulizing element is then actuated to nebulize the mixture in the removable cartridge **228.**

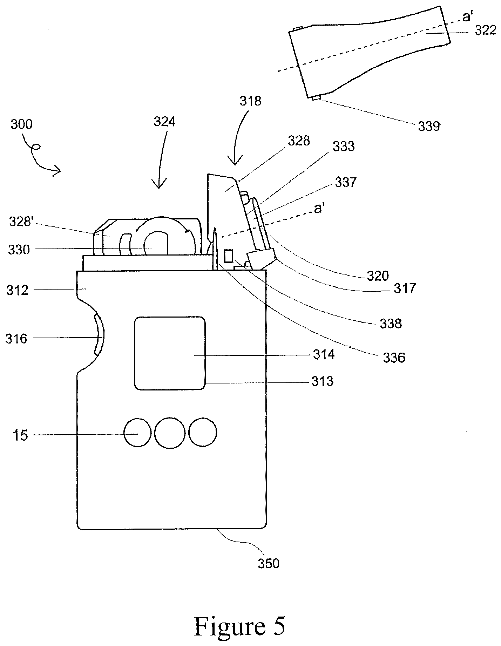

Figs. 4 and 5 show embodiments of a handheld nebulizer having the characteristic of performing nebulization simultaneously with a user's breathing. As shown in Fig. 4, the handheld nebulizer **200** includes most of the components in the handheld nebulizer **100** as shown in Figs. 1 and 2. The handheld nebulizer **200** includes a body **212** having a control assembly **213** and an actuation assembly which is in electrical communication with the control assembly **213.** The actuation assembly **217** is responsive to a user's breathing and includes actuation elements **237**, **237**'; a nebulizing chamber **218** accommodating a removable cartridge **228** for conducting nebulization, the removable cartridge **228** holds a mixture of a compound to be nebulized, wherein a nebulizing element **232** is affixed or detachably mounted to the removable cartridge **228**, and wherein the removable cartridge **228** is in electrical communication with the control assembly **213.**

In this embodiment, the actuation elements **237**, **237**' are arranged in the nebulizing chamber **218**, and positioned between the guiding nozzle **222** and the nebulizing element **232**. The actuation elements **237**, **237**' are in electrical communication with the control assembly **213**. When the user inhales via the guiding nozzle **222**, the actuation element **237** shifts to bias against the actuation element **237**' because of the airflow current generated from the user's inhalation. This completes an electrical circuit and an actuation signal is generated and transmitted to the control assembly **213.** On the other hand, when the user stops breathing or exhales, the actuation element **237** no longer touches the actuation element **237**', breaking the circuit, and the nebulization is thus stopped. In this embodiment, the guiding nozzle **222** is arranged with a one-way valve **239** on or in the nozzle wall. When the user exhales, the one-way valve **239** opens to discharge the exhaled air so as to reduce or avoid the exhaled air to come into contact with the actuation elements **237**, **237**' and/or to enter the nebulizing chamber **218.** When the user inhales or stops breathing, the one-way valve **239** is closed. The arrangement of the one-way valve **239** ensures the actuation elements **237**, **237**' function normally and react to the user's breathing in a sensitive manner. In one embodiment, the actuation element is between the one way valve and the exit of the guiding nozzle. The one-way valve can be of any design an ordinary skill person in the art can appreciate. For example, the guiding nozzle is arranged with an opening on its wall and a silicone film is arranged to cover the opening.

In an embodiment herein, the actuation element may be a sensor, for example but not limited to an infrared sensor and/or a flow sensor. The sensor may be installed in the nebulizing chamber, or attached to the guiding nozzle to detect the air current passing through the guiding nozzle so as to inform the control assembly and/or the nebulizing element to carry out the corresponding nebulization. When a user's inhalation is detected, a nebulization will be performed simultaneously. When no inhalation is detected, no nebulization will be performed.

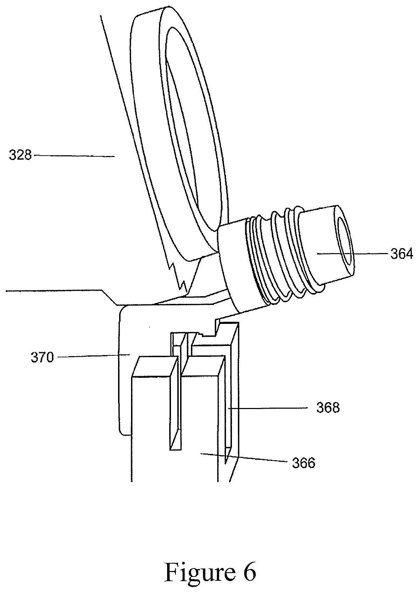

As shown in Fig. 5, the handheld nebulizer **300** makes use of a sensor **366** to detect a user's breathing for performing the corresponding nebulization. The handheld nebulizer **300** includes an actuation assembly **317** in the nebulizing chamber **318.** The actuation assembly **317** is positioned adjacent to the removable cartridge **328** and includes actuating elements, i.e. an infrared sensor **366** and a movable block **364** (as shown in Fig. 6). With reference to Fig. 6, the movable block **364** can be movably arranged in the recess **368** of the infrared sensor **366.** During operation, the infrared sensor **366** generates an infrared light beam and detects, in real time, whether the infrared light beam has passed through the recess. If no infrared light beam is detected, the infrared sensor **366** will send an actuation signal to the control assembly (see **313** in Fig. 5) which is operatively-connected with the infrared sensor **366.** When the infrared light beam is detected, the infrared sensor **366** will not send such a signal and/or will stop sending any actuation signal.

When the user inhales, a negative pressure is established in the guiding nozzle **322** and the nebulizing chamber **318.** The negative pressure drives the movable block **364** to move towards the user and the tab **370** therefore interrupts the infrared light beam within the infrared sensor **366.** The infrared sensor **366** then detects the changes immediately and sends an actuation signal to the control assembly to trigger the corresponding nebulization. The movable block **364** is designed to be particularly sensitive to pressure changes. When the user stops breathing, the movable block **364** returns to its initial position and allows the infrared beam light to pass through the recess **368.** The infrared sensor **366** detects the changes (i.e. detects the infrared light) and then stops sending the actuation signal (or sends a stop signal). The nebulization is thus stopped. When the user exhales, provided that a one-way valve **339** is arranged in the guiding nozzle **322**, the exhaled air is discharged through the one-way valve **339** without affecting the actuation assembly **317.**

In an embodiment herein, the guiding nozzle, **122, 222, 322** may be provided as a disposable form so as to further reduce the chance of contamination.

Based on the disclosure herein, the ordinary person skilled in the art is able to implement the one or more features of the present invention into any known structures of handheld nebulizer for obtaining a handheld nebulizer with substantially improved effects. For example, Chinese Patent No. 103143086B, Chinese Patent No.103143087B, Chinese Patent No.103143089B, Chinese Patent No.103143090B, Chinese Patent No.103143088B and Chinese Patent No.103170040B have already provided some possible structures of a handheld nebulizer. The disclosed structures in these publications cannot achieve the desired effects as described in the present invention. Accordingly, the present invention provides possible means and features to improve the existing handheld nebulizer.

It will be appreciated by persons skilled in the art that numerous variations and/or modifications may be made to the invention as shown in the specific embodiments without departing from the scope of the invention as defined by the appended claims.

Any reference to prior art contained herein is not to be taken as an admission that the information is common general knowledge, unless otherwise indicated.

## Claims

1. A handheld nebulizer (100, 200, 300) comprising:
- a body (112, 212) comprising a control assembly (113, 213, 313) and an actuation assembly (117, 217, 317), wherein the control assembly (113, 213, 313) is in electrical communication with the actuation assembly (117, 217, 317) for delivering nebulized particles of a compound to a subject;
- a removable cartridge (128, 228, 328) for holding a mixture of a compound to be nebulized into nebulized particles, wherein a nebulizing element (132, 232) is affixed or detachably mounted to the removable cartridge (128, 228, 328), and wherein the removable cartridge (128, 228, 328) is in electrical communication with the control assembly (113, 213, 313);
- a nebulizing chamber (118, 218, 318) for accommodating the removable cartridge (128, 228, 328) for conducting nebulization; and
- a heating element (136) for heating the mixture in the removable cartridge (128, 228, 328) before nebulization.

2. The handheld nebulizer according to claim 1, wherein the nebulizing chamber (118, 218, 318) comprises an outlet (120), and wherein the outlet (120) is in fluid communication with a guiding nozzle (122, 222, 322) connected to the nebulizing chamber (118, 218, 318) for guiding the flow of the nebulized particles.

3. The handheld nebulizer according to any one of claims 1 or 2, wherein the nebulized particles have an average size of 5µm or less than 5µm.

4. The handheld nebulizer according to any one of the preceding claims, further comprises a storage chamber (124) arranged to accommodate an additional removable cartridge (128, 228, 328).

5. The handheld nebulizer according to claim 1, wherein the nebulized particles delivered after the heating and nebulization have a suitable average temperature for the subject to inhale.

6. The handheld nebulizer according to any one of claims 1 or 5, wherein the heating element (136) is a heating coil, a heating plate, a copper tube, or a combination thereof.

7. The handheld nebulizer according to claim 5, wherein the average temperature is of from 25°C to 40°C.

8. The handheld nebulizer according to any preceding claims, further comprising an identification element (138) recognized by the control assembly (113, 213, 313) to determine the identity of the removable cartridge (128, 228, 328).

9. The handheld nebulizer according to claim 8, wherein the identification element (136) is integrated in to the removable cartridge (128, 228, 328) or is a separate element attached to the removable cartridge (128, 228, 328).

10. The handheld nebulizer according to claim 8 or 9, wherein the identification element (136) is an RFID tag, an electronic chip card, a printed label, a designed structure of the removable cartridge or a combination thereof.

11. The handheld nebulizer according to claim 8 or 9, wherein the control assembly (113, 213, 313) adjusts preset parameters of the nebulizer according to the identity of the removable cartridge (128, 228, 328).

12. The handheld nebulizer according to any one of the preceding claims, wherein the control assembly (113, 213, 313) comprises a display, a timer, a sensor, an input button, a circuit board, a database or a combination thereof.

13. The handheld nebulizer according to any one of the preceding claims, wherein the actuation assembly (117, 217, 317) is responsive to a user's breathing, said actuating assembly (117, 217, 317) comprises an actuating element (237, 237') for detecting a change in air current or air pressure.

14. The handheld nebulizer according to claim 13, wherein the nebulizing chamber comprises an outlet (120) in fluid communication with a guiding nozzle (122, 222, 322) connected to the nebulizing chamber (118, 218, 318), and wherein said guiding nozzle (122, 222, 322) further comprises a one-way valve (239, 339).

15. The handheld nebulizer according to any preceding claim, wherein the nebulizing element is an ultrasonic atomizer.

## Patentansprüche

1. Handgeführter Vernebler (100, 200, 300), der Folgendes umfasst:
- einen Körper (112, 212), der eine Steuerungsbaugruppe (113, 213, 313) und eine Betätigungsbaugruppe (117, 217, 317) umfasst, wobei die Steuerungsbaugruppe (113, 213, 313) zum Abgeben von vernebelten Teilchen einer Verbindung an ein Subjekt in elektrischer Verbindung mit der Betätigungsbaugruppe (117, 217, 317) steht,
- eine entfernbare Kartusche (128, 228, 328) zum Enthalten eines Gemischs einer Verbindung, die zum vernebelten Teilchen vernebelt werden soll, wobei ein Verneblungselement (132, 232) an der entfernbaren Kartusche (128, 228, 328) befestigt oder abnehmbar angebracht ist, und wobei die entfernbare Kartusche (128, 228, 328) in elektrischer Verbindung mit der Steuerungsbaugruppe (113, 213, 313) steht,
- eine Verneblungskammer (118, 218, 318) zum Aufnehmen der entfernbaren Kartusche (128, 228, 328) zum Durchführen der Verneblung und
- ein Heizelement (136) zum Erhitzen des Gemischs in der entfernbaren Kartusche (128, 228, 328) vor der Verneblung.

2. Handgeführter Vernebler nach Anspruch 1, wobei die Verneblungskammer (118, 218, 318) einen Auslass (120) umfasst, und wobei der Auslass (120) mit einer Leitdüse (122, 222, 322), die mit der Verneblungskammer (118, 218, 318) verbunden ist, zum Leiten des Stroms der vernebelten Teilchen in Fluidverbindung steht.

3. Handgeführter Vernebler nach einem der Ansprüche 1 oder 2, wobei die vernebelten Teilchen eine durchschnittliche Größe von 5 µm oder weniger als 5 µm aufweisen.

4. Handgeführter Vernebler nach einem der vorhergehenden Ansprüche, der ferner eine Speicherkammer (124) umfasst, die dafür angeordnet ist, eine zusätzliche entfernbare Kartusche (128, 228, 328) aufzunehmen.

5. Handgeführter Vernebler nach Anspruch 1, wobei die vernebelten Teilchen, die nach dem Erhitzen und der Verneblung abgegeben werden, eine geeignete durchschnittliche Temperatur, damit das Subjekt sie inhaliert, aufweisen.

6. Handgeführter Vernebler nach einem der Ansprüche 1 oder 5, wobei das Heizelement (136) eine Heizschlange, eine Heizplatte, eine Kupferröhre oder eine Kombination derselben ist.

7. Handgeführter Vernebler nach Anspruch 5, wobei die durchschnittliche Temperatur von 25 °C bis 40 °C beträgt.

8. Handgeführter Vernebler nach einem der vorhergehenden Ansprüche, der ferner ein Identifikationselement (138) umfasst, das durch die Steuerungsbaugruppe (113, 213, 313) erkannt wird, um die Identität der entfernbaren Kartusche (128, 228, 328) zu bestimmen.

9. Handgeführter Vernebler nach Anspruch 8, wobei das Identifikationselement (136) in die entfernbare Kartusche (128, 228, 328) integriert ist oder ein gesondertes Element ist, das an der entfernbaren Kartusche (128, 228, 328) befestigt ist.

10. Handgeführter Vernebler nach Anspruch 8 oder 9, wobei das Identifikationselement (136) ein RFID-Etikett, eine elektronische Chipkarte, ein gedrucktes Etikett, eine gestaltete Struktur der entfernbaren Kartusche oder eine Kombination derselben ist.

11. Handgeführter Vernebler nach Anspruch 8 oder 9, wobei die Steuerungsbaugruppe (113, 213, 313) voreingestellte Parameter des Verneblers entsprechend der Identität der entfernbaren Kartusche (128, 228, 328) einstellt.

12. Handgeführter Vernebler nach einem der vorhergehenden Ansprüche, wobei die Steuerungsbaugruppe (113, 213, 313) eine Anzeige, eine Zeitsteuerung, einen Sensor, einen Eingabeknopf, eine Leiterplatte, eine Datenbank oder eine Kombination derselben umfasst.

13. Handgeführter Vernebler nach einem der vorhergehenden Ansprüche, wobei die Betätigungsbaugruppe (117, 217, 317) auf den Atem eines Benutzers reagiert, wobei die Betätigungsbaugruppe (117, 217, 317) ein Betätigungselement (237, 237') zum Erkennen einer Veränderung bei einem Luftstrom oder Luftdruck umfasst.

14. Handgeführter Vernebler nach Anspruch 13, wobei die Verneblungskammer einen Auslass (120) in Fluidverbindung mit einer Leitdüse (122, 222, 322), die mit der Verneblungskammer (118, 218, 318) verbunden ist, umfasst, und wobei die Leitdüse (122, 222, 322) ferner ein Einwegventil (239, 339) umfasst.

15. Handgeführter Vernebler nach einem der vorhergehenden Ansprüche, wobei das Verneblungselement ein Ultraschall-Zerstäuber ist.

## Revendications

1. Nébuliseur à main (100, 200, 300), comprenant :
- un corps (112, 212) comprenant un ensemble de commande (113, 213, 313) et un ensemble d'actionnement (117, 217, 317), l'ensemble de commande (113, 213, 313) étant en communication électrique avec l'ensemble d'actionnement (117, 217, 317) pour administrer des particules nébulisées d'un composé à un sujet ;
- une cartouche amovible (128, 228, 328) pour contenir un mélange d'un composé devant être nébulisé en particules nébulisées, dans lequel un élément de nébulisation (132, 232) est fixé ou monté de manière détachable sur la cartouche amovible (128, 228, 3289), et dans lequel la cartouche amovible (128, 228, 328) est en communication électrique avec l'ensemble de commande (113, 213, 313) ;
- une chambre de nébulisation (118, 218, 318) pour recevoir la cartouche amovible (128, 228, 328) pour effectuer la nébulisation ; et
- un élément de chauffage (136) pour chauffer le mélange dans la cartouche amovible (128, 228, 328) avant la nébulisation.

2. Nébuliseur à main selon la revendication 1, dans lequel la chambre de nébulisation (118, 218, 318) comprend une sortie (120) et dans lequel la sortie (120) est en communication de fluide avec une buse de guidage (122, 222, 322) connectée à la chambre de nébulisation (118, 218, 318) pour guider l'écoulement des particules nébulisées.

3. Nébuliseur à main selon l'une quelconque des revendications 1 ou 2, dans lequel les particules nébulisées ont une dimension moyenne de 5 µm ou inférieure à 5 µm.

4. Nébuliseur à main selon l'une quelconque des revendications précédentes, comprenant en outre une chambre de stockage (124) configurée pour recevoir une cartouche amovible additionnelle (128, 228, 328).

5. Nébuliseur à main selon la revendication 1, dans lequel les particules nébulisées, administrées après le chauffage et la nébulisation, ont une température moyenne appropriée en vue d'une inhalation par le sujet.

6. Nébuliseur à main selon l'une quelconque des revendications 1 à 5, dans lequel l'élément de chauffage (136) est un serpentin de chauffage, une plaque chauffante, un tube en cuivre ou une combinaison de ceux-ci.

7. Nébuliseur à main selon la revendication 5, dans lequel la température moyenne est comprise entre 25°C et 40°C.

8. Nébuliseur à main selon l'une quelconque des revendications précédentes, comprenant en outre un élément d'identification (138) reconnu par l'ensemble de commande (113, 213, 313) pour déterminer l'identité de la cartouche amovible (128, 228, 328)

9. Nébuliseur à main selon la revendication 8, dans lequel l'élément d'indentification (136) est intégré dans la cartouche amovible (128, 228, 328) ou est constitué par un élément séparé fixé sur la cartouche amovible (128, 228, 328).

10. Nébuliseur à main selon les revendications 8 ou 9, dans lequel l'élément d'indentification (136) est une étiquette RFID, une carte à puce électronique, une étiquette imprimée, une structure de conception de la cartouche amovible ou une combinaison de ceux-ci.

11. Nébuliseur à main selon les revendications 8 ou 9, dans lequel l'ensemble de commande (113, 213, 313) ajuste des paramètres prédéfinis du nébuliseur en fonction de l'identité de la cartouche amovible (128, 228, 328).

12. Nébuliseur à main selon l'une quelconque des revendications précédentes, dans lequel l'ensemble de commande (113, 213, 313) comprend un affichage, une minuterie, un capteur, une touche d'entrée, une carte de circuit imprimé, une base de données ou une combinaison de ceux-ci

13. Nébuliseur à main selon l'une quelconque des revendications précédentes, dans lequel l'ensemble d'actionnement (117, 217, 317) est sensible à la respiration d'un utilisateur, ledit ensemble d'actionnement (117, 217, 317) comprenant un élément d'actionnement (237, 237') pour détecter un changement d'un courant d'air ou d'une pression de l'air.

14. Nébuliseur à main selon la revendication 13, dans lequel la chambre de nébulisation comprend une sortie (120) en communication de fluide avec une buse de guidage (122, 222, 322) connectée à la chambre de nébulisation (118, 218, 318), ladite buse de guidage (122, 222, 322) comprenant en outre une soupape unidirectionnelle (239, 339).

15. Nébuliseur à main selon l'une quelconque des revendications précédentes, dans lequel l'élément de nébulisation est un atomiseur à ultrasons.
